# DEMANDE DE BREVET EUROPEEN

(11) **EP 1 593 346 A1**
(43) Date de publication de la demande: **09.11.2005**
(21) Numéro de dépôt: 04370014.5
(22) Date de dépôt: 04.05.2004
(51) Int. Cl.: A61B 17/00

(54) **Tire-veine lumineux collimaté à linéaire d'utilisation opératif successivement réajustable**

(71) Demandeur: L'Echevin, Patrick, 62530 Hersin-Coupigny (FR); Berqué, Emmanuel, 59850 Nieppe (FR)
(72) Inventeur: L'Echevin, Patrick, 62530 Hersin-Coupigny (FR); Berqué, Emmanuel, 59850 Nieppe (FR); Decuypere, Dominique, 59800 Lille (FR)

(57) **Abrégé**

Tire-veine lumineux collimaté à linéaire d'utilisation opératif successivement réajustable est composé d'une fibre optique plastique monobloc (1) à diffusion axiale AA' **caractérisé en ce que** la fibre optique (1) est composée, d'un connecteur adaptatif (2) pour connecter tout câble d'émission de lumière froide de type connu pour transmettre cette lumière au collimateur optique (4) qui la concentre vers le coeur de la fibre (1), d'une bague tubulaire d'arrêt (5) solidaire de la fibre (1), d'une poignée amovible (6) pour coulisser le long de la fibre (1) selon une liaison pivot glissant ou fixe en fonction d'un moyen de commande (7) et, à son extrémité distale, d'une coupe en biseau (23) de sa section pour obtenir une diffusion axiale et ponctuellement radiale de ladite lumière.

L'invention se rapporte au domaine technique des éveinages saphènes internes et notamment à un tire-veine lumineux collimaté à linéaire d'utilisation opératif successivement réajustable destiné au repérage des veines par transillumination ainsi qu'à leurs ablations successives.

## Description

L'invention se rapporte au domaine technique des éveinages saphènes internes et notamment à un tire-veine lumineux collimaté à linéaire d'utilisation opératif successivement réajustable destiné au repérage des veines par transillumination ainsi qu'à leurs ablations successives.

Il existe différentes techniques d'éveinage qui sont actuellement :

L'exo-éveinage réalisé de différentes façons :
- par un tire-veine muni d'une boule qui « arrache » la veine et la décolle des tissus péri-veineux, cette technique est décrite dans le brevet N° FR 2.133.338 publiée le 30 octobre 1972, grâce à «un instrument pour le 'tringlage' chirurgical des veines en matière plastique synthétique».
- par des appareils d'exo-éveinage par anneaux dont l'action est voisine de celle du tire-veine.
- par des phlébectomies au crochet qui attire la veine par une mini incision et, par traction et dissection, l'extériorise pour la retirer par des incisions en nombre variable.

Et l'endo-éveinage qui assure le retournement en « doigt de gant » ou invagination de la veine, qui est retirée par traction à l'intérieur de la lumière vasculaire, cette méthode étant traumatisante pour les structures nerveuses péri-veineuses. Le même principe est appliqué lors des éveinages sur tiges.

La présente invention s'intéressera au mode d'exo-éveinage par tire-veine.

Il existe de manière connue divers dispositifs de repérage par éclairage interne ponctuel du corps de veine et/ou de sa périphérie externe sous ou à côté de celle-ci.

Ces dispositifs utilisent pour la plupart un conduit lumineux à fibre optique pour propager dans le corps d'un patient une source de lumière extérieure de manière à créer une transillumination de la lumière au travers des tissus.

La définition des ces dispositifs nous renseigne sur l'utilisation simultanée d'introduction de liquides de contraste visant à accentuer le rendu lumineux de la transillumination.

En effet, l'intensité du rendu lumineux de la transillumination détermine visuellement et surtout avec une précision proportionnelle le cheminement veineux principale de la saphène ainsi que la localisation des veines perforantes.

Rappelons que l'exo-éveinage de la veine saphène interne est pratiquée pour le traitement de ces dilatations pathologiques permanentes (varices) mais qu'il est nécessaire, voir obligatoire dans 70% des cas de manière connue, de prévoir la ligature des veines perforantes connectées sur la saphène.

En effet et selon ce même pourcentage, si tel n'est pas le cas, les veines perforantes disposées en vis-à-vis après l'éveinage de la saphène peuvent se reconnecter pour former de nouvelles varices locales. Ce risque récidive peut évidement nécessiter une ré-intervention chirurgicale.

De fait, il est primordiale d'obtenir une transillumination intense afin de pouvoir localiser précisément d'une part le cheminement principal d'une veine saphène mais également et surtout de pouvoir pratiquer un balisage de surface, en externe de la peau, du positionnement des veines perforantes afin de pouvoir les ligaturer après l'éveinage de la saphène.

Ces ligatures sont alors réalisées par de petites incisions locales qui ont été marquées avec précision sur la peau du patient lors dudit balisage.

Le problème ainsi posé fait état de la difficulté d'obtenir une transillumination suffisante en fonction des sources connues et présentes dans tout bloc d'intervention chirurgical. Ce qui explique la présence revendiquée d'introduction simultanée de liquide de contraste dans les canaux veineux.

De plus, rappelons que les sources lumineuses présentes dans les blocs chirurgicaux sont généralement utilisées pour des applications d'endoscopie et/ou de coelioscopie et sont classiquement d'origine artificielles selon une technologie halogène, iodures métallique ou xénon.

Ces sources lumineuses sont généralement intégrées dans un générateur de lumière sous la forme d'un appareil portable ou d'une borne fixe sur lesquels se connecte sur un câble de lumière dite froide.

Ce câble de lumière froide qui est en fait un conduit de multiples fibres optiques de verre selon une section d'environ 5 mm connectable selon deux types d'embouts connus et standard sur l'appareillage opératif tel qu'un endoscope par exemple.

Les deux embouts de type connus et standard disposent une bague de liaison à clipser pour l'un et à visser pour l'autre.

D'autre part et selon une méthode connue, la mise en oeuvre d'un tire-veine s'effectue par une incision de la veine saphène interne pratiquée à proximité de la cheville ainsi qu'au niveau inguinal.

Le câble d'étirement s'introduit alors de manière conventionnelle de la cheville pour ressortir de la saphène au niveau de l'aine.

Ce câble est équipé de façon solidaire par deux bagues tubulaires de butée d'un diamètre légèrement supérieur à celui dudit câble à chaque extrémité sur lesquelles viennent se solidariser une ogive au niveau de la cheville et une poignée de traction au niveau de l'aine.

C'est alors la traction de la poignée sur le câble d'étirement qui engendre l'entrée de l'ogive dans le corps du patient, suivant le cheminement de la veine saphène, et l'entraînant avec elle de part son diamètre important pour provoquer l'ablation de ladite saphène. La veine saphène prisonnière autour du câble est donc poussée par l'ogive pour alors ressortir du corps du patient par l'incision inguinale.

Les tire-veines de type connus possèdent un câble d'étirement d'une longueur fixe et standard déterminée selon une péréquation des linéaires de saphènes relatives aux tailles de jambes de l'ensemble des patients.

En pratique, ce linéaire par défaut revendique parfois une sortie du câble de plus de 20 cm au niveau de l'incision inguinale en exemple pour une personne de petite taille.

Cet état de fait est problématique pour le chirurgien car l'étirement en bout de bras de la poignée de traction n'est pas aisé voir périlleux à réaliser et source de fautes d'asepsies.

De plus, cette gymnastique manuelle peut parfois induire un défaut d'alignement mécanique de traction ce qui peut déformer l'incision inguinale au passage dudit câble.

En conclusion, la transillumination servant la visualisation en externe de veines saphènes internes ainsi qu'au balisage externe des veines perforantes revendique divers inconvénients liés à l'intensité du rendu lumineux ainsi qu'au linéaire du câble d'étirement.

La présente invention a pour but de remédier à ces inconvénients en proposant un tire-veine lumineux collimaté pour accentuer le rendu lumineux de la transillumination sans utilisation de liquide de contraste connectable sur tout type de câble à lumière froide standard et possédant un linéaire d'utilisation opératif successivement réajustable pour tout linéaire de veine.

Il est également à noter que la présente invention apportera une aide au chirurgien sans modifier le mode opératoire classique d'éveinage par tire-veine.

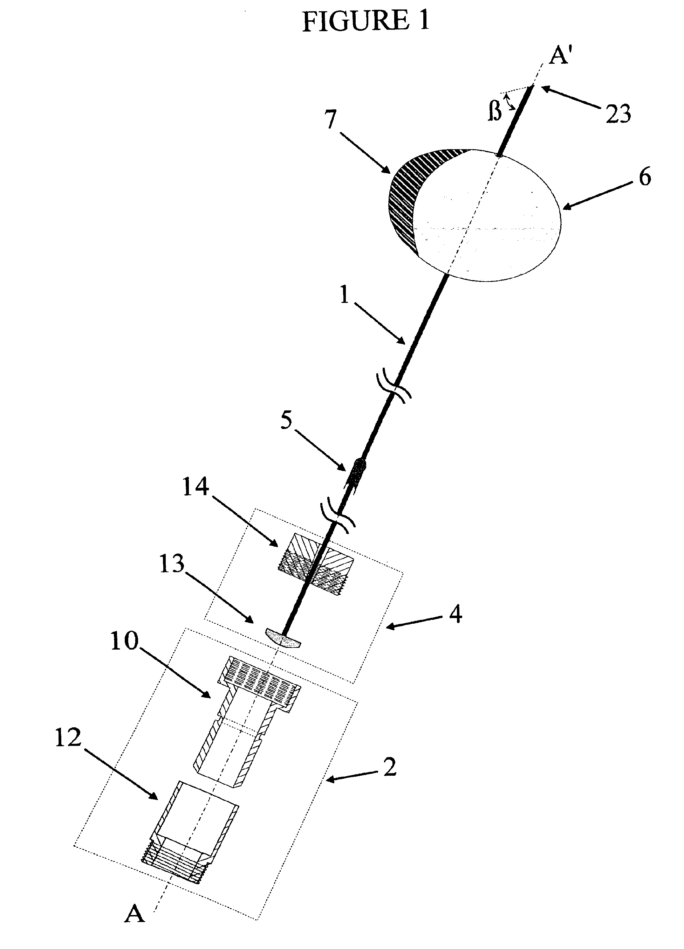

Pour ce faire et suivant la figure 1, le tire-veine lumineux collimaté à linéaire d'utilisation opératif successivement réajustable est composé d'une fibre optique plastique monobloc (1) à diffusion axiale AA' **caractérisé en ce que** la fibre optique (1) est composée, d'un connecteur adaptatif (2) pour connecter tout câble d'émission de lumière froide de type connu pour transmettre cette lumière au collimateur optique (4) qui la concentre vers le coeur de la fibre (1), d'une bague tubulaire d'arrêt (5) solidaire de la fibre (1), d'une poignée amovible (6) pour coulisser le long de la fibre (1) selon une liaison pivot glissant ou fixe en fonction d'un moyen de commande (7) et, à son extrémité distale, d'une coupe en biseau (23) de sa section pour obtenir une diffusion axiale et ponctuellement radiale de ladite lumière.

Le dispositif selon l'invention ainsi décrit dispose donc de nombreux avantages pour se connecter aisément sur tout câble de lumière froide équipant tout bloc opératoire pour collimater et amplifier la lumière disponible pour obtenir à l'extrémité distale de la fibre une diffusion axiale suffisante pour générer le guidage optique de la fibre dans la veine saphène lors de sa progression jusqu'à l'incision inguinale ainsi qu'une diffusion radiale suffisante pour générer la transillumination nécessaire au la visualisation des veines perforantes.

Cette particularité de diffusion est obtenue par la coupe en biseau de l'extrémité distale de la fibre optique et, de fait, le tire-veine selon l'invention est **caractérisé en ce que** la coupe en biseau (23) de la section de fibre optique (1) est défini selon un angle β compris entre 15° et 45° par rapport à son axe de symétrie AA' pour produire une réfraction des rayons lumineux générant la diffusion axiale et conjointement pour produire une réflexion des rayons lumineux générant la diffusion radiale de l'extrémité distale de la fibre (1).

La coupe en biseau de la fibre optique réalise donc un dioptre plan selon l'angle β qui est alors choisi judicieusement pour obtenir, en fonction des indices optiques de la fibre et du plasma sanguin, une réflexion lumineuse plus intense que la réfraction afin de produire une transillumination correcte et conjointement générer l'éclairage de l'avancée de la fibre optique dans la veine saphène. En pratique cet angle β est défini autour de 30° pour obtenir le double effet visuel optimal.

Il est de plus remarquable que ce double effet visuel permette de créer un spot lumineux lié à la caractéristique de réflexion lumineuse qui est visible par transillumination mais également que ce spot est directif par manipulation rotative de la fibre optique.

Cet effet de directivité est particulièrement intéressant pour « balayer » le champ visuel environnant et ainsi discerner les veines perforantes ou les cheminements parfois tortueux de la veine saphène souvent difficiles à traverser.

La fibre optique est avantageusement équipée d'un connecteur adaptatif permettant le branchement mécanique et optique du dispositif selon l'invention sur toute bague conventionnelle et standard équipant l'extrémité distale des câbles à lumière froide de coelioscopie ou d'endoscopie en exemple.

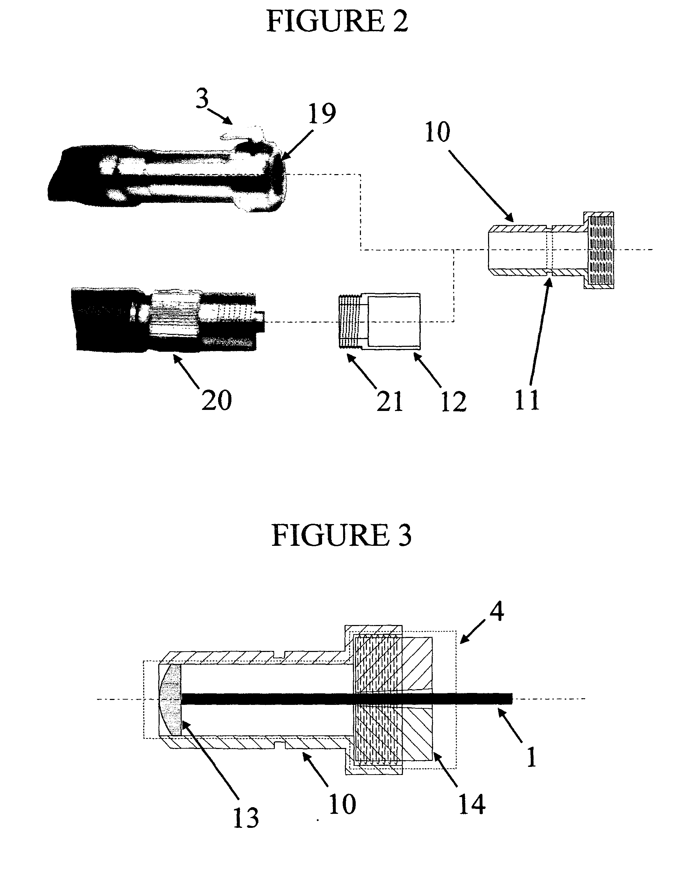

Pour ce faire, et suivant la figure 2 le tire-veine selon l'invention est **caractérisé en ce que** le connecteur adaptatif est composé d'une structure tubulaire (10) pourvue d'une gorge (11) pour être insérée dans toute bague à clipser (19) de câble à lumière froide et d'un adaptateur à vis (12) enclipsable sur ladite structure (10) pour être vissé sur toute bague à visser (20) de câble à lumière froide.

La structure tubulaire (10) du connecteur adaptatif connecte donc sur son diamètre extérieur dans l'intérieur de toute bague à clipser (19), le maintient et le jeu mécanique de l'assemblage est solutionné par une goupille mobile (3) présente sur toute bague à clipser qui entre alors dans la gorge (11) de ladite structure tubulaire (10).

L'adaptateur à vis (12) se connecte donc sur la structure tubulaire (10) de la même manière qu'une bague à clipser (19) d'un câble de lumière froide pour présenter un pas de vis (21) servant à visser toute bague à visser (20) de câble à lumière froide.

Ce connecteur adaptatif est donc en mesure de se connecter indifféremment sur une bague à clipser et/ou une bague à visser conventionnelle ce qui délivre une universalité de branchement du dispositif selon l'invention et engendre de fait une facilité de mise en oeuvre.

La lumière ainsi émise par ces câbles à lumière froide est donc dirigée par le connecteur adaptatif vers un collimateur pour la concentrer vers le coeur de la fibre optique.

En effet, l'augmentation maximale du rendu lumineux de la transillumination permettant d'éviter l'utilisation de liquide de contraste s'obtient également par la concentration collimatée des rayons lumineux provenant des fibres optiques verre de tout câble à lumière froide conventionnel vers le coeur de la fibre optique plastique monobloc.

En outre, le conduit des fibres optiques verre revendiquent un diamètre bien supérieur, de l'ordre de quelques 5 millimètres par rapport aux quelques dizaines de microns du diamètre du coeur de la fibre optique plastique monobloc. En exemple d'utilisation d'une fibre optique plastique monobloc de 2 mm de diamètre.

La problématique est dont de conduire à une concentration collimatée d'une section standard de quelques millimètres vers une section de quelques microns.

Pour ce faire et suivant la figure 3 en coupe, le tire-veine selon l'invention est **caractérisé en ce que** le collimateur optique (4) possède un moyen (14) de guidage axial et de fixation mécanique de ladite fibre optique (1) sur la structure tubulaire (10) et comporte au moins une lentille convergente (13) d'une distance focale la plus courte possible pour faire converger la lumière vers le coeur de la fibre optique (1).

En effet, le collimateur tel que défini, dispose judicieusement d'au moins une lentille convergente pour focaliser et collimater les rayons lumineux issus des câbles à lumière froide vers le coeur de la fibre optique plastique monobloc.

En pratique, la lentille utilisée est du type convexe/plan ou biconvexe associée à une concave/plan. Cette particularité permet d'obtenir une distance focale courte afin de créer un cercle de moindre diffusion le plus petit possible. Rappelons à ce sujet que le cercle de moindre diffusion est le plus petit cercle de concentration lumineuse.

De fait, plus la distance focale est courte, plus l'intensité lumineuse est concentrée dans une petite surface, ceci afin de viser le coeur de la fibre optique.

Le moyen de guidage axial et de fixation mécanique de la fibre optique permet alors d'assembler définitivement la fibre et son collimateur au connecteur adaptatif suivant son axe de symétrie.

Cette caractéristique particulièrement intéressante permet d'exploiter au maximum les sources de lumières artificielles présentent dans tout bloc chirurgical.

La fibre optique plastique ainsi fortement éclairante à son extrémité distale va permettre de discerner le suivi interne du cheminement de la veine aussi tortueux soit-il et plus encore, de pouvoir visualiser les veines perforantes pour les baliser en surface sur la peau du patient.

Il est également remarquable que le mode opératoire conventionnel d'utilisation d'un tire-veine classique (sans lumière) est conservé de part l'introduction de l'extrémité distale éclairante de la fibre, qui représente ici le câble d'étirement, au niveau de la cheville pour la faire transiter dans la saphène jusqu'à sa sortie extérieur du corps au niveau inguinal.

La sortie de la fibre au niveau inguinal doit s'effectuer jusqu'à ce que la bague tubulaire d'arrêt solidaire de la fibre optique arrive à proximité immédiate de l'incision au niveau de la cheville.

Cette bague tubulaire va supporter une ogive décrite dans l'une des revendications suivantes pour provoquer l'arrachage de la veine saphène.

En remarque, il est à noter que la distance entre la bague tubulaire d'arrêt et le collimateur optique doit être d'environ 50 cm afin de parer aux fautes d'asepsie entre l'ogive et le collimateur.

La partie de fibre optique ressortie de l'incision inguinale va alors être équipée d'une poignée amovible qui possède la particularité de coulisser le long de ladite fibre selon un mouvement de pivot glissant mais également avoir la particularité de pouvoir se fixer et devenir solidaire de ladite fibre en fonction d'une commande manuelle présente sur ladite poignée.

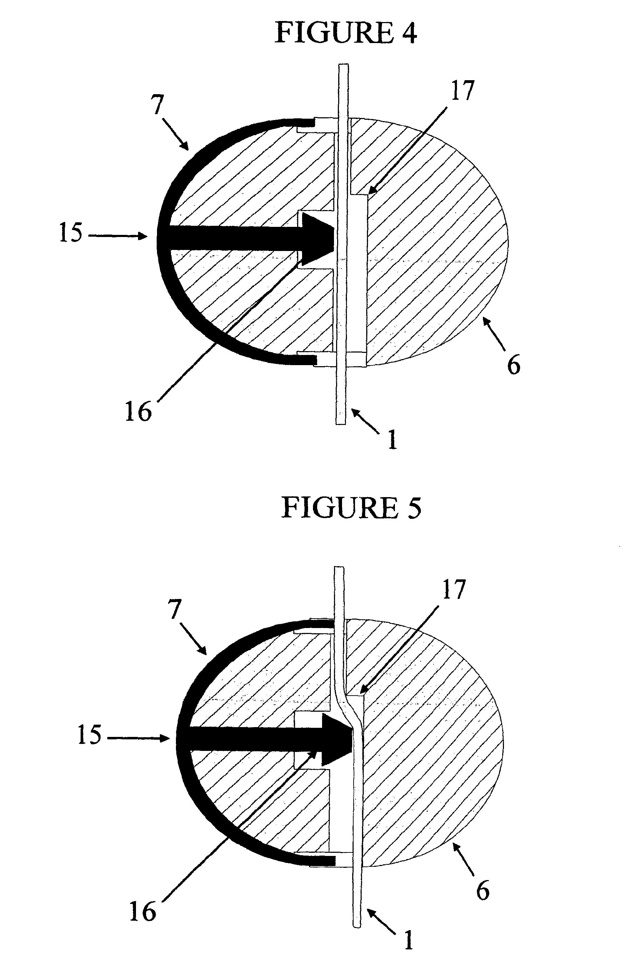

Pour ce faire et selon les figures 4 et 5, le tire-veine selon l'invention est **caractérisé en ce que** le moyen de commande (7) de la poignée amovible (6) est réalisé par un bouton poussoir (15) pour générer, lors de l'absence d'une poussée, une liaison en pivot glissant de la poignée amovible (6) sur la fibre optique (1), et pour générer, lors d'une action manuelle de poussée, l'avancée d'un piston (16) pour contraindre la fibre optique (1) à se désaxer en s'enfonçant dans une gorge d'arrêt (17) pour ainsi produire une liaison mécanique fixe par rapport à la poignée amovible (6).

Cette caractéristique particulièrement intéressante permet d'endiguer le phénomène de longueur fixe et standard de câble d'étirement déterminée selon la péréquation des linéaires de saphènes relatives aux tailles de jambes de l'ensemble des patients, évitant de fait l'obtention d'une sortie de plus de 20 cm au niveau de l'incision inguinale et les inconvénients qui en découlent.

En effet, l'ogive effectuant l'arrachage étant placée sur la bague tubulaire d'arrêt et amenée au plus près de l'incision au niveau de la cheville va conditionner la coulisse en pivot glissant de la poignée amovible le long de la fibre optique jusqu'à l'approche de l'incision inguinale

De fait, le linéaire d'utilisation opératif de fibre optique faisant office de câble d'étirement sera ajuster selon le linéaire dédiée à la saphène pour chaque cas anatomique à traiter.

Le chirurgien n'aura donc plus qu'à agir manuellement sur la poignée amovible pour la rendre fixe et solidaire de la fibre optique et commencer à tirer pour produire l'éveinage.

Plus encore, la poignée amovible peut successivement coulisser ou être fixe par rapport à la fibre optique selon ledit moyen de commande ce qui permet alors au chirurgien de fixer la poignée pour effectuer une première traction et arrachage puis, de faire à nouveau coulisser, selon le mouvement de pivot glissant, la poignée au plus proche de l'incision inguinal et de réitérer une traction et arrachage et ainsi de suite.

Cette particularité liée à la fonction de commande de la poignée amovible permet donc judicieusement de régler le linéaire de façon dédiée et de travailler avec une distance de sortie de la fibre optique à l'incision inguinale constante et donc plus précise à souhait du chirurgien.

Pour que le mode opératoire conventionnel d'utilisation d'un tire-veine classique (sans lumière) soit conservé et en fonction de la mise en place de l'ogive sur la bague tubulaire d'arrêt, il est logiquement nécessaire que la fibre optique et l'ogive soit mécaniquement séparée du collimateur et de son connecteur adaptatif lors du commencement de la phase de traction et d'entrée de l'ogive dans le corps.

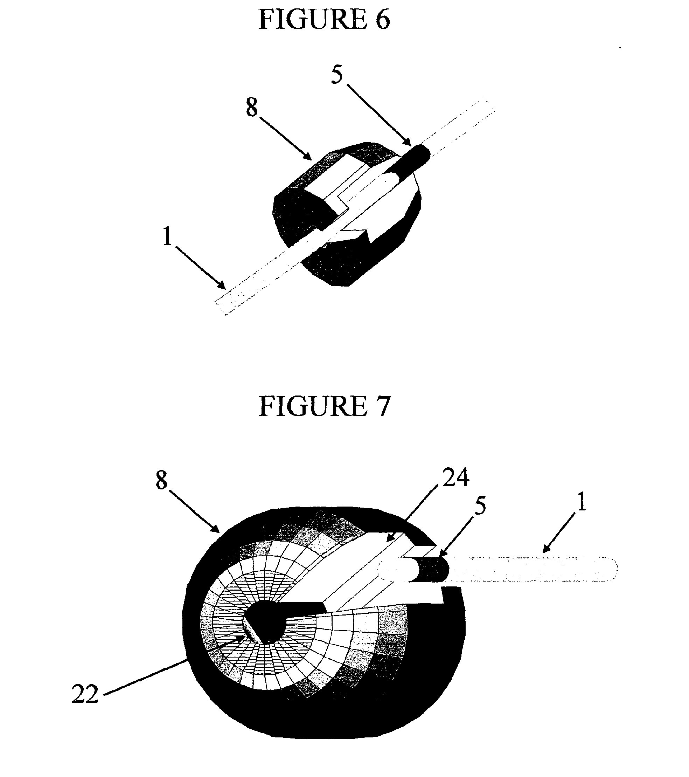

De fait et selon la figure 6, le tire-veine selon l'invention est **caractérisé en ce que** la bague tubulaire d'arrêt (5) supporte et fixe une bague cylindrique amovible en ogive (8) qui intègre, dans sa partie d'ogive, un moyen de découpe de la fibre optique (1) généré conjointement à l'action de mise en place de la bague cylindrique amovible en ogive (8) sur la bague tubulaire d'arrêt (5).

D'autres part et selon la figure 7, le tire-veine selon l'invention est **caractérisé en que** le moyen de découpe de la fibre optique (1) est constitué d'au moins une lame métallique coupante (22) perpendiculaire à la gorge d'insertion (24) de l'ogive (8) pour entailler légèrement la section la fibre optique (1) insérée dans la gorge de l'ogive (8).

Ainsi, la phase de mise en place de la bague cylindrique en ogive sur la bague tubulaire d'arrêt provoque la liaison mécanique fixe de l'ogive par rapport à la fibre optique et engendre simultanément une entaille latérale de la fibre optique. Cette seule entaille permet alors de casser manuellement la fibre optique sans effort et provoque alors la séparation mécaniquement de l'ogive liée à la fibre optique de l'ensemble formé par le collimateur et son connecteur adaptatif.

De fait, l'ogive n'est plus alors retenue et peut entrer dans le corps du patient à la manière d'un tire-veine classique selon un mode opératoire conventionnel connu des chirurgiens.

Afin d'optimiser encore un peu plus la collimation lumineuse et afin d'endiguer un effet de chauffe propre à certaine sources artificielles utilisées, la ou les lentilles constituant le collimateur optique se définissent comme filtrant les longueurs d'ondes susceptibles de réduire un échauffement potentiel de l'extrémité distale de la fibre optique dans le corps du patient.

A cette fin, le tire-veine selon l'invention est **caractérisé en ce qu'au** moins une lentille convergente (13) possède un revêtement de surface traité contre la transmission de lumière infrarouge.

De cette manière, même s'il s'agit de sources et de conduit à lumière froide, le spectre de la lumière infrarouge ne sera pas transmit au coeur de la fibre optique par le collimateur.

De fait, le trajet de la fibre optique dans la veine saphène ne peut provoquer un échauffement important lors de la visualisation par transillumination.

L'introduction et notamment le cheminement mécanique de la fibre optique dans la veine saphène doivent se réaliser en évitant les accroches de l'extrémité distale éclairante contre les parois internes de ladite veine.

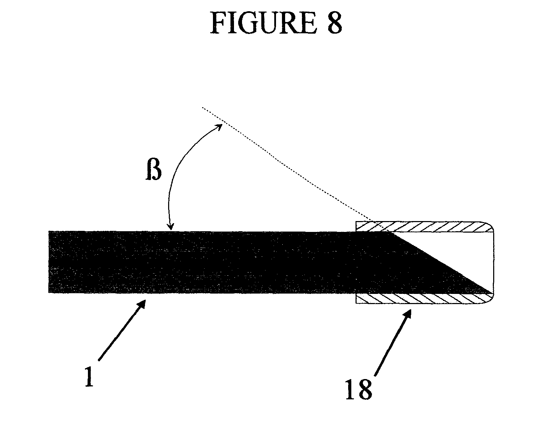

Pour ce faire et suivant la figure 8, le tire-veine selon l'invention est **caractérisé en ce que** la fibre optique (1) est pourvue, à son extrémité distale, d'une bague d'adoucissement (18) et/ou d'un adoucissement de la périphérie du bord de sa section distale pour minimiser tout angle vif.

De cette manière le risque de déchirement interne de la veine saphène au passage de la fibre optique est alors minimisé. En exemple d'un mode de fabrication, certaines pièces du tire-veine selon l'invention sont réalisées suivant une technologie par injection plastique.

## Revendications

1. Tire-veine lumineux collimaté à linéaire d'utilisation opératif successivement réajustable est composé d'une fibre optique plastique monobloc (1) à diffusion axiale AA' **caractérisé en ce que** la fibre optique (1) est composée, d'un connecteur adaptatif (2) pour connecter tout câble d'émission de lumière froide de type connu pour transmettre cette lumière au collimateur optique (4) qui la concentre vers le coeur de la fibre (1), d'une bague tubulaire d'arrêt (5) solidaire de la fibre (1), d'une poignée amovible (6) pour coulisser le long de la fibre (1) selon une liaison pivot glissant ou fixe en fonction d'un moyen de commande (7) et, à son extrémité distale, d'une coupe en biseau (23) de sa section pour obtenir une diffusion axiale et ponctuellement radiale de ladite lumière.

2. Tire-veine suivant la revendication 1, **caractérisé en ce que** la coupe en biseau (23) de la section de fibre optique (1) est défini selon un angle β compris entre 15° et 45° par rapport à son axe de symétrie AA' pour produire une réfraction des rayons lumineux générant la diffusion axiale et conjointement pour produire une réflexion des rayons lumineux générant la diffusion radiale de l'extrémité distale de la fibre (1).

3. Tire-veine suivant la revendication 1, **caractérisé en ce que** le connecteur adaptatif est composé d'une structure tubulaire (10) pourvue d'une gorge (11) pour être insérée dans toute bague à clipser (19) de câble à lumière froide et d'un adaptateur à vis (12) enclipsable sur ladite structure (10) pour être vissé sur toute bague à visser (20) de câble à lumière froide.

4. Tire-veine suivant la revendication 1 et 3, **caractérisé en ce que** le collimateur optique (4) possède un moyen (14) de guidage axial et de fixation mécanique de ladite fibre optique (1) sur la structure tubulaire (10) et comporte au moins une lentille convergente (13) d'une distance focale la plus courte possible pour faire converger la lumière vers le coeur de la fibre optique (1).

5. Tire-veine suivant l'une quelconque des revendications précédentes, **caractérisé en ce que** le moyen de commande (7) de la poignée amovible (6) est réalisé par un bouton poussoir (15) pour générer, lors de l'absence d'une poussée, une liaison en pivot glissant de la poignée amovible (6) sur la fibre optique (1), et pour générer, lors d'une action manuelle de poussée, l'avancée d'un piston (16) pour contraindre la fibre optique (1) à se désaxer en s'enfonçant dans une gorge d'arrêt (17) pour ainsi produire une liaison mécanique fixe par rapport à la poignée amovible (6).

6. Tire-veine suivant l'une quelconque des revendications précédentes, **caractérisé en ce que** la bague tubulaire d'arrêt (5) supporte et fixe une bague cylindrique amovible en ogive (8) qui intègre, dans sa partie d'ogive, un moyen de découpe de la fibre optique (1) généré conjointement à l'action de mise en place de la bague cylindrique amovible en ogive (8) sur la bague tubulaire d'arrêt (5).

7. Tire-veine suivant l'une quelconque des revendications précédentes, **caractérisé en que** le moyen de découpe de la fibre optique (1) est constitué d'au moins une lame métallique coupante (22) perpendiculaire à la gorge d'insertion (24) de l'ogive (8) pour entailler légèrement la section la fibre optique (1) insérée dans la gorge de l'ogive (8).

8. Tire-veine suivant l'une quelconque des revendications précédentes, **caractérisé en ce qu'**au moins une lentille (13) possède un revêtement de surface traité contre la transmission de lumière infrarouge.

9. Tire-veine suivant l'une quelconque des revendications précédentes, **caractérisé en ce que** la fibre optique (1) est pourvue, à son extrémité distale, d'une bague d'adoucissement (18) et/ou d'un adoucissement de la périphérie du bord de sa section distale pour minimiser tout angle vif.
